# EUROPEAN PATENT SPECIFICATION

(11) **EP 2 496 217 B1**
(45) Date of publication and mention of the grant of the patent: **27.03.2019**
(21) Application number: 09786999.4
(22) Date of filing: 24.08.2009
(51) Int. Cl.: A61K 9/20, A61K 31/245

(54) **DIRECT COMPRESSION TABLETS OF OTILONIUM**
DIREKTVERPRESSUNG VON OTILONIUM
COMPRIMÉS D'OTILONIUM OBTENUS PAR COMPRESSION DIRECTE

(43) Date of publication of application: 12.09.2012
(73) Proprietor: Abdi Ibrahim Ilac Sanayi ve Ticaret Anonim Sirketi, Istanbul (TR)
(72) Inventor: FARSHI, Farhad, Bahcesehir 34555 Istanbul (TR); AVCI, Recep, Bahcesehir 34555 Istanbul (TR); KANDEMIRER, Urun, Bahcesehir 34555 Istanbul (TR); SOYLEMEZ, Serdar, Bahcesehir 34555 Istanbul (TR); KOC, Fikret, Bahcesehir 34555 Istanbul (TR)
(86) International application number: PCT/IB2009/053701
(87) International publication number: WO 2011/024028

(56) References cited:
- WO-A1-00/76478
- WO-A1-2004/009073
- WO-A1-2010/092436
- CN-A- 101 053 562
- DATABASE WPI Week 200823 Thomson Scientific, London, GB; AN 2008-D16834 XP002577566 & CN 101 053 562 A (BEIJING DEZHONG WANQUAN MEDICINE TECHNOL) 17 October 2007 (2007-10-17)

## Description

This invention is related to direct compression of otilonium or its pharmaceutically acceptable salt having perfect powder flowability, good tablet weight distribution and no sticking to the dies or punches of tablet press.

Otilonium bromide is used as an antispasmodic for treating spastic painful conditions of the distal section of the intestinal tract, including IBS(irritable bowel syndrome). It is used for the treatment of irritable bowel, pain and spasm of the distal enteric tract. Experimental studies exhibits that otilonium bromide inhibits both baseline and chemically or physically stimulated gastrointestinal motility. Clinical studies have confirmed otilonium bromide as a spasmolytic drug with a good tolerability profile.

Spasmoctyl® includes otilonium bromide which is on the market as a reference drug.

Spasmoctyl® tablet contains lactose, starch, sodium starch glycolate, magnesium stearate,hypromellose (hydroxypropylmethylcellulose),titanium dioxide and polyethylene glycol.

Spasmoctyl® tablets are prepared by using of granulation technics. In the course of preparing Spasmoctyl® tablets, the blended product is granulated prior to pressing (M. Blanco et al., Development and validation of a near infrared method for the analytical control of a pharmaceutical preparation in three steps of the manufacturing process, Fresenius J Anal Chem, 2000, 368 :534-539, page 535).

Concerning qualitative composition of Spasmoctyl® 40 mg Film Coated Tablets; starch is the only excipient that can be used as a binder. In tablet formulations technology, freshly prepared starch paste at 50°-70°C is used in tablet granulations as a binder (Handbook of Pharmaceutical Excipients,Fifth Edition,Edited by Raymond C Rowe et al) thus it is understood that in Spasmoctyl® starch is used and it points out that tablet formulation is prepared through using of granulation methods. Meanwhile selection of the quantity should be chosen very carefully because the granulation with starch paste may cause some problems such as poor granule friability, tablet friability, capping, low hardness, disintegration rate, and drug dissolution rate and this kind of manufacturing process with starch paste is much more difficult and requires experienced staff. On the other hand such manufacturing process takes more times and more expenditures. CN 101053562A discloses a pharmaceutical composition of otilonium bromide obtained by wet granulation with ethanol and most preferably containing 43.33% of lactose, 20.67% of microcrystalline cellulose, 8% of crospovidone, 1.3% magnesium stearate.

In pharmaceutical technology, it is well known that if possible, direct compression is prefered rather than granulation. In direct compression, the active and the excipients are blended together and compressed directly without extra processing, such as granulation. Direct compression is the most effective and favorable manufacturing process for the production of solid dosage forms. Thus manufacturers would prefer to use direct compression techniques since It is advantageous overs granulation. Direct compression technique has quick processing and cost advantages.

One aspect of this disclosure relates to a tablet composition comprising otilonium or its pharmaceutically acceptable salts and spray-dried lactose as diluent in the range of from 20 % to 85 %, copovidone as binder in the range of from 2 % to 10 %, sodium starch glycolate as disintegrant in the range of from 2 % to 10 % , colloidal silicone dioxide as glidant in the range of from 0.1 % to 1, magnesium stearate as lubricant in the range of from 0.25 % to 5 by weight of the tablet core, wherein the tablet is prepared by direct compression. Another aspect of this disclosure relates to a process according to claim 6 for the preparation of said tablet. It is the object of the present disclosure that direct compression pharmacetical compositions of otilonium bromide having eligible granule friability, tablet friability, hardness, disintegration rate, and drug dissolution rate are obtained.

In formulations, a direct comperssion agent or mixtures of agents are used. Direct compression agents are, but not limited to, pregelatinised starches, polyvinylpyrrolidone, methylcellulose, microcrystalline cellulose, sucrose, lactose, dextrose, sorbitol, mannitol, lactitol,xylitol, modified calcium salt, granulated corn starch, modified rice starch, compressible sugars such as Destab™,dextrates such as Emdex ® dicalcium phosphate, hydroxypropylcellulose , methylcellulose , hydroxypropylmethylcellulose, polyethylene glycol, amylose,anhydrous calcium hydrogen phosphate, calcium sulphate,tribasic calcium phosphate, dibasic calcium phosphate, low-crystallinity powdered cellulose,silicified microcrystalline cellulose,chitin,chitosan hydrochloride,copovidone,croscarmellose sodium,dextrose, anhydrous lactose,anhydrous alpha lactose,anhydrous beta lactose,agglomerated lactose, spray-dried lactose, maltodextrin, mixtures thereof and the like.

Spray dried type of lactose monohydrate is used in the present disclosure.

Mixtures and/or co-processed diuents which are suitable for direct compression can also be used such as anhydrous lactose- anhydrous lactitol, calcium sulphate-microcrystalline cellulose, lactose-cellulose, lactose-starch, lactose-povidone,Sucrose-maltodextrin coprecipitate and the like.

According to this invention, the pharmaceutical compositions further comprise pharmaceutically acceptable excipient or excipients selected from the group of diluents, disintegrants, binders, lubricants, glidants, mixtures thereof and the like.

When formulating direct compression tablets, the choice of direct compression (DC) binder is extremely critical. It must fulfill certain requirements: good binding functionality and powder flowability are essential. Another functionality of DC binders is their compressibility under pressure. Binders are, but not limited to, polyvinylpy-rolidone, copovidone, starches such as pregellatinized starch or plain starch, cellulose derivatives such as hydroxypropylmethylcellulose ,ethylcellulose, hydroxypropylcellulose and carboxymethylcellulose and their salts, gelatine, acacia, agar,alginic acid, carbomer, ceratonia, chitosan, dextrates,dextrin,glycerol dibehenate,guar gum,hypromellose, inulin,magnesium aluminum silicate,maltodextrin,poloxamer,polycarbophil,polydextrose, polyethylene oxide,polymethacrylates,sodium alginate, sucrose, hydrogenated vegetable oil, mixtures thereof and the like. Copovidone (vinylpyrrolidone-vinyl acetate copolymer) is used in the present disclosure. With copovidone, accelerated and long term stability results did not show any incompatibility at the final product.

Glidants are preferably selected from the group consisting of colloidal silicon dioxide, precipitated silica and pyrogenic silica, talc and aluminium silicate, tribasic calcium phosphate,calcium stearate, powdered cellulose,magnesium oxide,magnesium silicate,magnesium trisilicate,starch, talc, mixtures thereof and the like. During the development of the product, in order to improve flowability of the final mixture before compression colloidal silicone dioxide is used . Accelerated and long term stability results did not show any incompatibility at the final product.

Disintegrants are , but not limited to, modified starches, croscarmallose sodium, carboxymethylcellulose calcium, sodium starch glycolate ,crospovidone, alginic acid, calcium alginate,microcrystalline cellulose,powdered cellulose,chitosan,colloidal silicon dioxide,crospovidone,guar gum,low-substituted hydroxypropylcellulose,hydroxypropyl starch,magnesium aluminum silicate,methylcellulose,polacrilin potassium,sodium alginate,starch,pregelatinised starch,mixtures thereof and the like. Sodium starch glycolate is used in the present disclosure.

Lubricants are, but not limited to, magnesium stearate,calcium stearate,hydrogenated castor oil,glyceryl behenate,glyceryl monostearate,glyceryl palmi-tostearate,leucine,mineral oil, light mineral oil, myristic acid,palmitic acid,polyethylene glycol,potassium benzoate,sodium benzoate,sodium lauryl sulfate,sodium stearyl fumarate,stearic acid,talc, hydrogenated vegetable oil,zinc stearate, magnesium lauryl sulphate,sodium stearyl fumarate, polyethylene glycol, stearic acid, colloidal silicon dioxide or mixtures thereof. The lubricant used in the present disclosure is magnesium stearate.

Diluents are, but not limited to, anhydrous lactose, lactose monohydrate, modified lactose, dibasic calcium phosphate, tribasic calcium phosphate, microcrystalline cellulose, silicified microcrystalline cellulose, powdered cellulose, maize starch, prege-latinized starch, calcium carbonate, sucrose, glucose, dextrates, dextrins, dextrose, fructose, lactitol, mannitol, sorbitol, starch, and mixtures thereof.

According to a preferred embodiment of the present invention, the tablet cores are coated . Coating is preferably film coating. Coating agents are, but not limited to, sugars, hydroxypropyl methylcellulose, hydroxypropylcellulose, methylcellulose, ethyl cellulose, polyvinyl alcohol, sodium carboxymethyl cellulose, coatings based on methacrylic acid and its esters, such as Eudragit ®, mixtures thereof and the like. Coating may further contain an excipient or excipients, for example, titanium oxide, talc, ferric oxide, polyethylene glycol and the like. As a coating agent multifunction ingredient such as Opadry ® II 85F18422 can also be used.

According to this invention eligible flowability and compressibility is determined with direct compression.

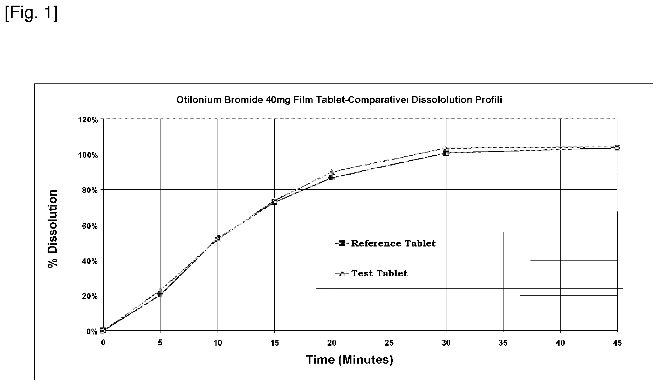

Dissolution profiles of reference tablets (Spasmoctyl®) and test tablets should be same or identical in desired dissolution mediums. Desired dissolution profile means that in targeted dissolution mediums f2 (similarity factor) value should be at least 50 to 100 when compared to the reference Spasmoctyl® dissolution profile. According to this invention direct compression tablets of otilonium (called as test tablet) is in desired ranges of f2 value (Figure 1) .

On the other hand this invention includes a preparation method for direct compression formulations of otilonium. This method comprising steps of a. Direct compression agent, otilonium bromide and binder are installed into a container and mixed, b. Disintegrant and pre-sieved glidant are installed into container onto powder mixture prepared at step a and mixed , c. Pre-sieved lubricant is installed into container onto powder mixture prepared at step b and mixed , d. The final mixture is compressed in a tablet press at 30-70 N average hardness, e. a coating agent or mixtures of coating agents are added into purified water and stirred, f. Core tablets are installed into a coating pan and coated with the coating suspension prepared at step e.

The following reference example is provided and is not intended to limit the scope of the invention.

### Reference Example 1

### [Table 1]

**[Table]**

| Formulation Table | |
|---|---|
| **Excipients** | **% by weight of the tablet core** |
| Direct Compression Agent | 50.5 |
| Binder | 5 |
| Disintegrant | 2 |
| Glidant | 0.5 |
| Lubricant | 2 |

## Claims

1. A tablet composition comprising otilonium or its pharmaceutically acceptable salts and spray-dried lactose as diluent in the range of from 20 % to 85 %, copovidone as binder in the range of from 2 % to 10 %, sodium starch glycolate as disintegrant in the range of from 2 % to 10 % , colloidal silicone dioxide as glidant in the range of from 0.1 % to 1, magnesium stearate as lubricant in the range of from 0.25 % to 5 by weight of the tablet core, wherein the tablet is prepared by direct compression.

2. According to claim 1, pharmaceutically acceptable salt of otilonium is bromide.

3. According to claim 1, tablet is at 30-70 N average hardness.

4. According to claim 1, tablet is coated.

5. According to claim 4, agents used in coating are selected from the group consisting of sugars, hydroxypropyl methylcellulose, hydroxypropylcellulose methylcellulose, ethyl cellulose, polyvinyl alcohol, sodium carboxymethyl cellulose, methacrylic acid and its esters (Eudragit ®), mixtures thereof.

6. A process for the preparation of a tablet according to any one of the preceding claims comprising the steps of
(a) mixing spray-dried lactose, otilonium bromide and copovidone;
(b) mixing the powdery mixture of sodium starch glycolate and pre- sieved colloidal silicone dioxide with the powder mixture obtained in step (a);
(c) mixing pre-sieved magnesium stearate with the powder mixture obtained in step (b);
(d) subjecting the final mixture obtained in step (c) to compression;
(e) coating of tablets obtained in step (d).

## Patentansprüche

1. Tablettenzusammensetzung, umfassend Otilonium oder dessen pharmazeutisch verträgliche Salze und sprühgetrocknete Laktose als Verdünnungsmittel in einem Bereich von 20 bis 85 Gew.-%, Copovidon als Bindemittel in einem Bereich von 2 bis 10 Gew.-%, Natriumstärkeglykolat als Sprengmittel in einem Bereich von 2 bis 10 Gew.-%, kolloidales Silikondioxid als Gleitmittel in einem Bereich von 0,1 bis 1 Gew.-%, Magnesiumstearat als Schmiermittel in einem Bereich von 0,25 bis 5 Gew.-% des Tablettenkerns, wobei die Tablette durch direkte Kompression hergestellt wird.

2. Gemäß Anspruch 1, ist das pharmazeutisch verträgliche Salz von Otilonium Bromid.

3. Gemäß Anspruch 1, hat die Tablette eine durchschnittliche Härte von 30-70 N.

4. Gemäß Anspruch 1, wird die Tablette beschichtet.

5. Gemäß Anspruch 4, werden die zur Beschichtung verwendeten Mittel ausgewählt aus der Gruppe bestehend aus Zucker, Hydroxypropylmethylcellulose, Hydroxypropylcellulose, Methylcellulose, Ethylcellulose, Polyvinylalkohol, Natriumcarboxymethylcellulose, Methacrylsäure und deren Ester (Eudragit®), sowie Mischungen davon.

6. Verfahren zur Herstellung einer Tablette nach einem der vorhergehenden Ansprüche umfassend die Schritte von
(a) Mischen von sprühgetrockneter Lactose, Otiloniumbromid und Copovidon;
(b) Vermischen der pulverförmigen Mischung aus Natriumstärkeglykolat und vorgesiebtem kolloidalem Silikon mit der in Schritt (a) erhaltenen Pulvermischung;
(c) Vermischen von vorgesiebtem Magnesiumstearat mit der in Schritt (b) erhaltenen Pulvermischung;
(d) Unterziehen von in Schritt (c) erhaltenen Endmischung einer Kompression;
(e) Beschichten von in Schritt (d) erhaltenen Tabletten.

## Revendications

1. Une composition de comprimé comprenant otilonium ou ses sels pharmaceutiquement acceptables et lactose séché par pulvérisation en tant que diluant dans la plage de 20% à 85%, copovidone en tant que liant dans la plage de 2% à 10%, glycolate d'amidon sodique en tant que désintégrant dans la plage de 2% à 10%, dioxyde de silice colloïdale en tant qu'agent glissant dans la plage de 0.1% à 1%, stéarate de magnésium en tant que lubrifiant dans la plage de 0.25% à 5% par rapport au poids de comprimé-noyau, où le comprimé est préparé par compression directe.

2. Selon la revendication 1, le sel pharmaceutiquement acceptable est bromure.

3. Selon la revendication 1, le comprimé a une dureté moyenne de 30-70 N.

4. Selon la revendication 1, le comprimé est enrobé.

5. Selon la revendication 4, les agents utilisés pour enrobage sont sélectionné parmi le groupe constitué des sucres, hydroxypropylméthylcellulose, hydroxypropylcellulose, méthylcellulose, éthylcellulose, alcool polyvinylique, carboxyméthylcellulose sodique, acide méthacrylique et ses esters (Eudragit®), mélange de celles-ci.

6. Un procède de préparation d'un comprimé selon l'une quelconque des revendications précédentes, qui comprend les étapes de:
(a) mélanger lactose séché par pulvérisation, bromure d'otilonium et copovidone;
(b) mélanger le mélange pulvérulent de glycolate d'amidon sodique et de dioxyde de silice colloïdale pré-tamisé avec le mélange pulvérulent obtenu dans l'étape (a);
(c) mélanger stéarate de magnésium pré-tamisé avec le mélange pulvérulent obtenu dans l'étape (b);
(d) compression du mélange final obtenu dans l'étape (c);
(e) enrobage des comprimés obtenus dans l'étape (d).
